# EUROPÄISCHE PATENTSCHRIFT

(11) **EP 1 455 190 B1**
(45) Veröffentlichungstag und Bekanntmachung des Hinweises auf die Patenterteilung: **25.10.2006**
(21) Anmeldenummer: 04003422.5
(22) Anmeldetag: 16.02.2004
(51) Int. Cl.: G01N 33/74, C12Q 1/68, G01N 33/68, A61K 38/00, A61K 48/00

(54) **Verfahren zur Bestimmung des hormonellen Effekts von Substanzen mittels der Interaktion zwischen dem Androgenrezeptor und Ewing Sarkoma Protein.**
Method for determining the hormonal effect of substances via the interaction of the Androgenreceptor with Ewing Sarcoma Protein.
Procédé de détermination des effets hormonaux des substances par l'intermédiaire de l'interaction entre le récepteur d'androgène et la protéine du Ewing Sarcome

(30) Priorität: 04.03.2003 DE 10309280; 25.04.2003 US 465692 P
(43) Veröffentlichungstag der Anmeldung: 08.09.2004
(73) Patentinhaber: Schering Aktiengesellschaft, 13353 Berlin (DE)
(72) Erfinder: Obendorf, Maik, Dr., 99423 Weimar (DE); Wolf, Siegmund, Dr., 07639 Klosterlausnitz (DE)
(74) Vertreter: Geyer, Fehners & Partner

(56) Entgegenhaltungen:
- WO-A-02/36621
- DE-A- 10 121 710
- ARAYA NATSUMI ET AL: "Cooperative interaction of EWS with CREB-binding protein selectively activates hepatocyte nuclear factor 4-mediated transcription" J. BIOL. CHEM.;JOURNAL OF BIOLOGICAL CHEMISTRY FEB 14 2003, Bd. 278, Nr. 7, 14. Februar 2003 (2003-02-14), Seiten 5427-5432, XP002277045
- FELSCH JASON S ET AL: "Tyrosine kinase Pyk2 mediates G-protein-coupled receptor regulation of the Ewing sarcoma RNA-binding protein EWS" CURRENT BIOLOGY, Bd. 9, Nr. 9, 6. Mai 1999 (1999-05-06), Seiten 485-488, XP002277046 ISSN: 0960-9822
- MALTAIS ANNIE ET AL: "The AF2 domain of the orphan nuclear receptor TEC is essential for the transcriptional activity of the oncogenic fusion protein EWS/TEC" CANCER LETTERS, Bd. 183, Nr. 1, 8. September 2002 (2002-09-08), Seiten 87-94, XP008029880 ISSN: 0304-3835
- VERMEULEN A ET AL: "Diagnosis of hypogonadism in the aging male" AGING MALE 2002 UNITED KINGDOM, Bd. 5, Nr. 3, 2002, Seiten 170-176, XP008032194 ISSN: 1368-5538

## Beschreibung

Die Erfindung betrifft ein Verfahren zur Bestimmung des hormonellen Effektes von Substanzen sowie ein Verfahren zur Bestimmung von Störungen im Co-Modulationsmechanismus des nukleären Androgenrezeptors. Darüberhinaus bezieht sich die Erfindung auf die Verwendung von Ewing Sarcoma Protein (EWS) oder von EWS Derivaten welche die Aminosäuren 319 bis 656 der in Sequenz ID No. 1 beschriebenen Sequenz aufweisen sowie von Nukleinsäuren, welche dafür kodieren.

Bei der Beurteilung von Substanzen für eine mögliche pharmazeutische Anwendung ist es allgemein üblich, diese Substanzen auf eine eventuelle hormonelle Wirkung, insbesondere auf eine möglicherweise vorhandene androgene oder antiandrogene Aktivität, zu prüfen. Bei der Verabreichung pharmakologisch wirksamer Substanzen sind Kenntnisse über deren hormonelle Effekte, insbesondere androgene oder antiandrogene Effekte, zur Beurteilung etwaiger Nebenwirkungen, wichtig. Zur Prüfung der hormonellen Wirkung von Substanzen kommen beispielsweise Verfahren zum Einsatz, bei denen die Fähigkeit der Substanzen gemessen wird, an die Hormonrezeptoren zu binden und deren Transkriptionsaktivität zu aktivieren.

Kenntnisse über hormonelle Effekte von Substanzen sind aber nicht nur bei potentiellen Pharmaka von Interesse, sondern auch bei nicht-pharmazeutischen Substanzen, da von vielen, in der Umwelt vorhandenen Substanzen angenommen wird, dass sie bei Teilen der Bevölkerung eine androgene oder antiandrogene bzw. estrogene oder antiestrogene Aktivität aufweisen können. Möglicherweise wird dadurch eine unerwünschte, schädliche Wirkung hervorgerufen.

Eine besondere Schwierigkeit liegt in der Identifizierung und Charakterisierung von Effekten auf die von Steroidhormonen vermittelten Wirkungen, da die Signalkaskaden und - Netzwerke, die letztlich die hormonvermittelte Transkriptionsregulation steuern, hier besonders komplex ausgestaltet sind. Der Grund dafür liegt in der sehr ähnlichen Ausgestaltung der DNA-Zielsequenzen, an welche die verschiedenen Steroidhormonrezeptoren nach Ligandenaktivierung binden. Diese bedingt, dass die nukleären Rezeptoren zur Auslösung einer gezielten Antwort auf die Interaktion mit speziellen Co-Faktoren angewiesen sind, die unter anderem die Spezifität der rezeptorvermittelten Transkriptionsaktivierung verstärken.

Zur Identifizierung von Substanzen, welche auf bestimmte hormoninduzierte Signalwege einwirken, sind daher Testsysteme und -Verfahren notwendig, die gezielt die Funktion einzelner Komponenten des zellulären Signalnetzwerkes zur Vermittlung steroidaler Effekte erfassen können.

Es besteht somit ein Bedarf an einem Verfahren, mit dem in zuverlässiger, empfindlicher, einfacher, kostengünstiger und schneller Weise eine Aussage über den hormonellen Effekt von Substanzen getroffen werden kann. Die bisher bekannten Verfahren genügen dem nicht.

Der vorliegenden Erfindung liegt deshalb die Aufgabe zu Grunde, ein Verfahren bereitzustellen, mit dem in zuverlässiger, empfindlicher, einfacher, kostengünstiger und schneller Weise eine Aussage über den hormonellen Effekt der zu testenden Substanzen getroffen werden kann.

Diese Aufgabe wird erfindungsgemäß gelöst durch ein Verfahren zur Bestimmung des hormonellen Effektes von Substanzen mit den Schritten Kontaktieren von Ewing Sarcoma Protein (EWS) oder eines die Aminosäuren 319 bis 656 der in Seq. ID No. 1 beschriebenen Sequenz aufweisendes Derivat davon mit dem Androgenrezeptor und einer Testsubstanz, und
a. Bestimmung des Einflusses der Testsubstanz auf die Bindung von EWS oder dessen Derivat und dem Androgenrezeptor, oder
b. Bestimmung des Einflusses der Testsubstanz auf die Liganden-induzierte Aktivität des Androgenrezeptors.

Als Derivat eines Proteins bzw. Polypeptids wird im Rahmen der vorliegenden Erfindung jede, z.B. durch Aminosäure-Deletion, Substitution, Insertion, Inversion, Addition oder Austausch erhaltene Variante des Proteins bzw. Polypeptids verstanden. Hierbei sind solche Derivate bevorzugt, die die Fähigkeit des unveränderten Proteins/Potypeptides, z.B. die Aktivität anderer Proteine zu beeiflussen oder diese zumindest zu binden, behalten haben (funktionelle Derivate).

Die Erfindung beruht auf der überraschenden Erkenntnis, dass das Ewing Sarkom Protein sowie davon abgeleitete Derivate umfassend die Aminosäuren 319 bis 656 der in Seq. ID No 1 beschriebenen Sequenz (im folgenden als EWS bezeichnet) die Fähigkeit haben, mit dem Androgenrezeptor zu interagieren und dessen Aktivität zu modulieren. Araya et al (JBC, 2003, vol. 278, 5427-5432 offenbart die Interaktion zwischen EWS und dem nukleären Rezeptor HNF4 alpha.

Die Superfamilie der nukleären Rezeptoren (NRs), zu der mehr als 50 verschiedene Proteine gehören, ist eine Gruppe verwandter Transkriptionsfaktoren, die die Transkription des jeweiligen Zielgens als Reaktion auf spezifische Liganden, z. B. Hormone, steuern. Die Familie kann nach bestimmten Charakteristika, wie z.B. Dimerisationsstatus, Art des Liganden oder Struktur des DNA-Reaktionselements, in mehrere Subfamilien unterteilt werden (Beato et al., 2000, Human Reproduct. Update, 6, 225-236). Charakteristisches Merkmal der NRs ist die übereinstimmende Struktur der funktionellen Domäne (mit den Bezeichnungen A bis F) mit einer stark variablen, nur schwach konservierten N-terminalen Region mit autonomer konstitutiver Aktivierungsfunktion (AF-1), einer stark konservierten DNA-Bindungsdomäne (DBD), die für die Erkennung von speziellen DNA-Reaktionselementen verantwortlich ist und aus zwei Zinkfinger-Motiven besteht, einer variablen Schamierdomäne und einer konservierten multifunktionalen C-terminalen Ligandenbindungsdomäne (LBD) mit Dimerisations- und Liganden-abhängiger Transaktivierungsfunktion (AF-2). Im Anschluss daran folgt die am weitesten C-terminal gelegene Region, deren Funktion nicht bekannt ist und die bei Rezeptoren wie z.B. PR (Progesteron-Rezeptor), PPAR (Peroxisomproliferator-aktivierter Rezeptor) und RXR (Retinoid-X-Rezeptor) fehlt (Mangelsdorf & Evans, 1995; Cell, 83, 841-850; Robyr et al., 2000, Mol. Endocrinol., 14, 329-347). Für einige NRs (z.B. den Androgen-Rezeptor (AR)) wurde nachgewiesen, dass die N-terminale Region in der Lage ist, mit der C-terminalen Region zu interagieren (Brinkmann et al., 1999, J. Steroid Biochem. and Mol. Biol., 69, 307-313). Steroidhormonrezeptoren wie z.B. Estrogen- (ER), Progesteron- (PR), Glukokortikoid-(GR), Mineralokortikoid- (MR) und Androgenrezeptoren (AR) binden steroidale Liganden, die sich von Pregnenolon ableiten, wie die Progestine, die Estrogene, die Glukokortikoide und die Mineralokortikoide, sowie Androgene. Die Ligandenbindung aktiviert den Rezeptor und steuert die Expression entsprechender Zielgene.

EWS ist bekannt als Proto-Onkogen des Ewing-Sarkoms und anderer Neoplasien wie z.B. der Klarzellsarkome von Sehnen und Aponeurosen, der klein- und rundzelligen desmoplastischen Intraabdominaltumoren sowie der extraskeletalen Chondrosarkome (Delattre, O., Zucman, J., Plougastel, B., Desmaze, C., Melot, T., Peter, M., Kovar, H., Joubert, 1., de Jong, P., Rouleau, G., Aurias, A., and Thomas, G., 1992, Nature 359: 162-165, Zucman, J., Delattre, O., Desmaze, C., Epstein, A. L., Stenman, G., Speleman, F., Fletchers, C.D., Aurias, A., and Thomas, G; 1993, Nature Genet. 4: 341-345, Gerald, W. L, Rosai, J. and Ladanyi, M., 1995, Proc. Natl. Acad. Sci. USA 92: 1028-1032, Laballe, Y., Zucman, J., Stenman, G., Kindblom, L.G., Knight, J., Turc-Carel, C., Dockhom-Dwomiszak, B., Mandahl, N., Desmaze, C., Peter, M., Aurias, A., Delattre, O., and Tho-mas, G., 1995, Hum. Mol. Genet. 4: 2219-2226). Bei all diesen Tumoren wird der EWS-Genlocus umarrangiert, so dass das Aminosäuren-Ende (N-Terminus) des Proteins mit einer DNA-Bindungsdomäne von FLI1, ERG1, ATF1 oder WT1 verschmilzt. Dieses N-terminale Ende des fusionierenden Proteins enthält die EWS-Exone 1-7 oder 1-8 oder 1-9. Liegt die Bruchstelle zwischen Exon 7 und Exon 8, weist der EWS-Anteil des durch die Fusion entstandenen Proteins keine Übereinstimmungen mit der hier definierten Androgenrezeptorbindungsdomäne auf. Liegt dagegen die Bruchstelle zwischen den Exonen 8 und 9 oder 9 und 10, stimmen nur 5 bzw. 20 Aminosäuren der beiden onkogenen EWS-Fusionsproteine mit dem EWS-Anteil überein, der die Androgenrezeptorbindungsdomäne enthält. Daraus ist zu schließen, dass die umarrangierten EWS-Fusionsproteine die Fähigkeit zur Bindung an Androgenrezeptoren verloren haben.

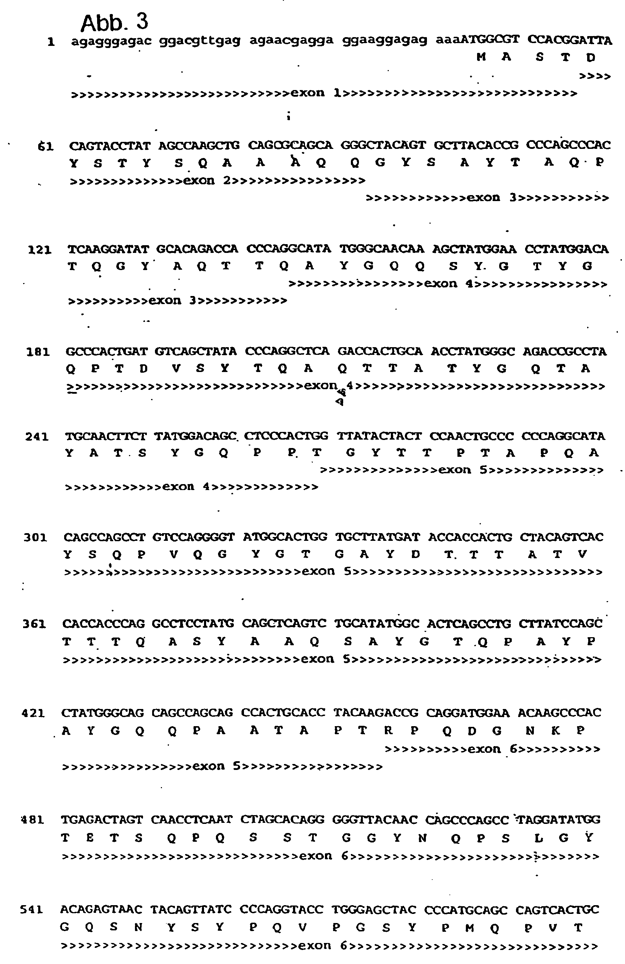

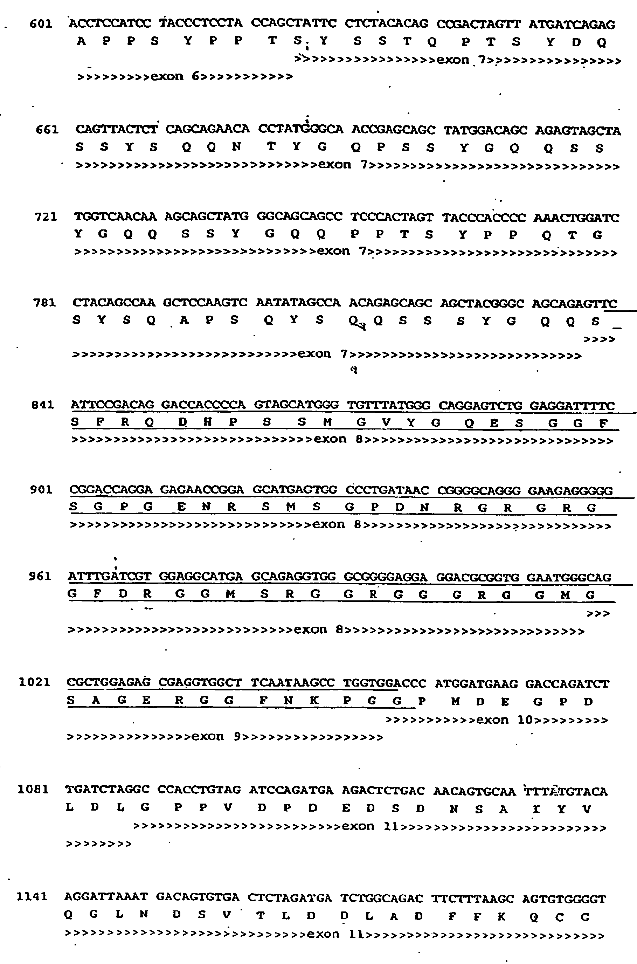

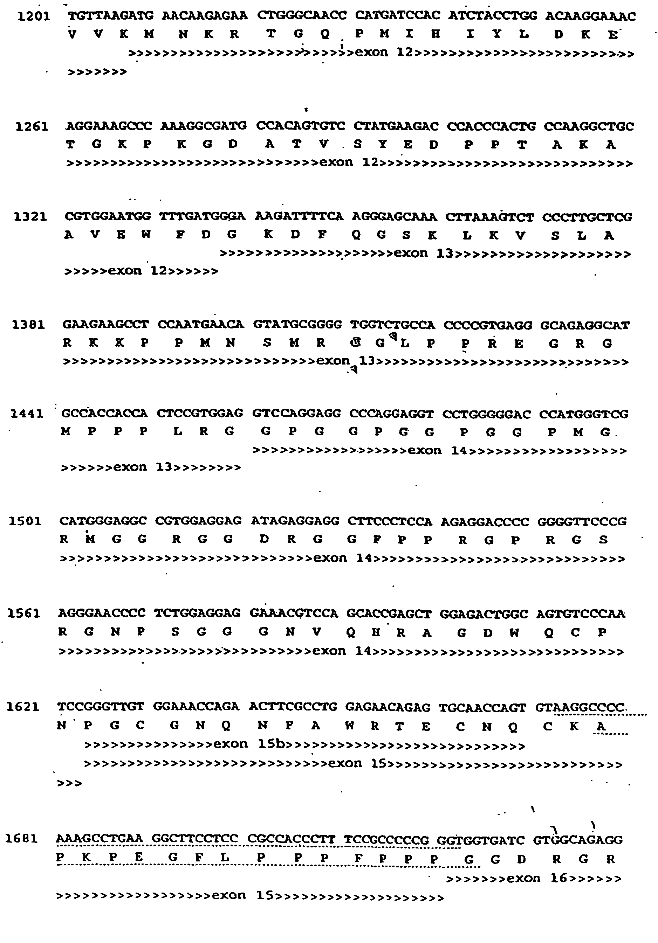

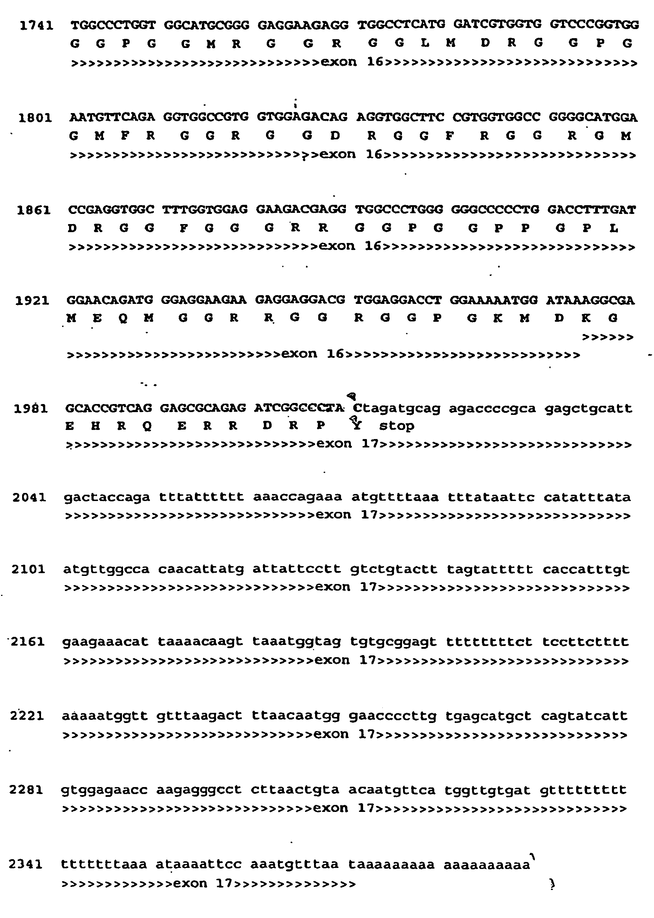

Bei der Analyse von Thymus-RNA mittels RT-PCR wurde eine EWS-Variante (EWS1-c), bei der 17 Aminosäuren fehlten (Abbildung 3), gefunden. Offensichtlich handelt es sich hierbei um eine Splice-Variante, da alle notwendigen Konsensus-Sequenzen an den Nahtstellen zwischen Intronen und Exonen vorhanden waren. Das Ergebnis war eine Verkürzung von Exon 15 (Exon 15b). Im Stand der Technik sind darüber hinaus weitere Splice-Varianten bekannt. Eine (Ohno, T., Ouchida, M., Lee, L., Gatalica, Z., Rao, V.N., and Reddy, E.S., 1994, Oncogene 9: 3087-3097) stellt ein um 200 bp verkürztes EWS-Transkript (EWS1-b) dar und wurde in ruhenden oder durch Phytohämagglutinin (PHA) stimulierten Lymphozyten gefunden. Dem EWS1-b fehlen die Exone 8 und 9. Eine andere Variante (Melot, T., Dauphinot, L., Sevenet, N., Radvanyi, F., Delattre, O. (2001), Eur. J. Biochem. 268, 3483-3489) enthält ein zusätzliches Exon 4' zwsischen den Exonen 4 und 5 und ist als gehirnspezifische Isoform bezeichnet.

EWS gehört zu einer Gruppe RNA-bindender Proteine, denen im Stand der Technik eine Implikation in Prozesse der RNA-Synthese bzw. -Prozessierung zugeschrieben wird, daneben war über die physiologische Funktion von somatischem wildtyp EWS bislang jedoch nur wenig bekannt. Insbesondere war im Stand der Technik nicht bekannt, dass EWS die Fähigkeit besitzt, nukleäre Rezeptoren zu binden und deren Aktivität zu modulieren, wodurch es der Klasse der NR-Co-Modulatoren zuzuordnen ist.

Ein *E.coli* Stamm mit der Bezeichnung *Escherichia* coli EWS-10 CMX wurde unter der Nr. DSM 15417 am 24. Janaruar 2003 bei der Deutschen Sammlung von Mikroorganismen und Zellkulturen GmbH (DSMZ) hinterlegt. *Escherichia* coli EWS-10 CMX enthält die full length-EWS-cDNA, die im erfindungsgemäßen Verfahren eingesetzt werden kann.

Die sog. Co-Modulatoren sind eine Klasse von Proteinen, die bei der Aktivierung (Co-Aktivatoren) bzw. Repression (Co-Repressoren) der Gentranskription als Brückenmoleküle zwischen dem Transkriptionsinitiationskomplex und den NRs dienen (McKenna et al., 1999, Endocr. Rev., 20, 321-347). Ein Co-Aktivator muss fähig sein, die Rezeptorfunktion zu verstärken und in Anwesenheit eines Agonisten mit der Aktivierungsdomäne von NRs direkt zu interagieren. Er muss auch mit dem basalen Transkriptionsapparat interagieren, und schließlich darf er nicht selbst die basale Transkriptionsaktivität verstärken. Die meisten Co-Modulatoren interagieren mit Hilfe eines oder mehrerer LXXLL-Motiv(en) (NR-Boxes) mit der AF-2-Domäne von NRs, jedoch wurden auch einige Co-Modulatoren beschrieben, die mit anderen NR-Regionen interagieren (Ding et al., 1998, Mol. Endocrinol., 12, 302-313). Ferner wurden viele Co-Modulatoren identifiziert, die in ähnlicher Weise mit mehreren verschiedenen NRs interagieren.

Das erfindungsgemäße Verfahren kann sowohl *in vitro* (also z.B. als rein biochemischer oder biophysikalischer Assay, in Lösung oder in geeigneten soliden Matrizes, etc.) als auch teilweise oder gänzlich im zellulären System ablaufen. Dem Fachmann sind solche unterschiedlichen Testsysteme hinlänglich bekannt.

Vorzugsweise läuft mindestens einer der Verfahrensschritte im zellulären System ab, da die Effekte der steroidvermittelten Transkriptionsaktivierung im physiologischen Kontext der Zelle besonders gut darstellbar sind. Demzufolge eignen sich für die Erfindung insbesondere eukaryontische Zellen, wobei sowohl primäre Zellen als auch etablierte Zellinien zum Einsatz kommen können. Die Verwendung etablierter Zelllinien erlaubt eine besonders gute Reproduzierbarkeit und Kostengünstigkeit, die Verwendung primärer Zellen vermeidet dagegen weitgehend durch Mutation und klonale Selektion bedingte Zellkultur-Artefakte. Besonders geeignet sind Prostatazellen, Nervenzellen, Gliazellen, Fibroblasten, Blutzellen, Osteoblasten, Osteoklasten, Hepatozyten, Epithelzellen oder Muskelzellen.

Der hier bestimmte (d.h. identifizierte, quantifizierte oder charakterisierte) hormonelle Effekt kann sowohl aktivatorischer als auch inhibitorischer Natur sein und neben der Einwirkung auf die Rezeptor-Co-Modulator-Bindung auch jeden anderen Schritt der NR-Aktion beziehen, z.B. die Liganden-induzierte Transaktivierung aber auch Kemlokalisierung des NR.

Gemäß einer bevorzugten Ausführungsform umfasst das erfindungsgemäße Verfahren die folgenden Schritte:
a. Zunächst werden Zellen, die EWS oder ein die Aminosäuren 319 bis 656 der in Seq. ID No.1 beschriebenen Sequenz aufweisendes Derivat davon und den Androgenrezeptor exprimieren, der zu testenden Substanz ausgesetzt.
b. Es folgt dann die Bestimmung des Einflusses der Substanz auf die Interaktion zwischen dem Rezeptor und EWS oder dessen obengenannten Derivats durch Messung der Protein-Protein-Interaktion oder der Protein-Protein-DNA-Interaktion.

Die Expression einer oder beider der miteinander interagierenden Komponenten (EWS/Derivat einerseits und NR/Derivat andererseits) kann in der Zelle von Natur aus oder Infolge transienter oder stabiler Transfektion mit geeigneten Expressionsvektoren erfolgen. Die Auswahl geeigneter Zelltypen und ggf. Vektorsysteme stellt eine dem zuständigen Fachmann geläufige Massnahme dar. Zur Expression im eukaryontischen System eignen sich beispielsweise pCMX oder pSG5 als Vektoren.

Die Messung der Protein-Protein-Interaktion zwischen Rezeptor bzw. Derivat und EWS bzw. Derivat oder der Protein-Protein-DNA-Interaktion der vorgenannten Komponenten mit der DNA-Zielsequenz erfolgt durch dem Fachmann bekannte Massnahmen. Hierzu eignen sich beispielsweise Techniken wie das Two-Hybrid-System, Co-Immunpräzipitation, GST-Pull-down-Assays, FRET-Analaysen und ABCD-Assays bzw. Gelretardationsassays zur Analyse von Protein-Protein-DNA Interaktionen.

Eine bevorzugte Ausführungsform des erfindungsgemäßen Verfahrens umfasst die Schritte:
a. Zellen, die EWS oder ein die Aminosäuren 319 bis 656 der in Seq. ID No.1 beschriebenen Sequenz aufweisendes Derivat davon und den Androgenrezeptor exprimieren und mit einem Reportergenkonstrukt transfiziert sind, werden dem Liganden des Androgenrezeptors und der zu testenden Substanz ausgesetzt,
b. Bestimmung der Transkriptionsaktivität des Androgenrezeptors durch Messung der Reportergenaktivität und
c. Vergleich mit der Transkriptionsaktivität bei Durchführung der Schritte a und b in Abwesenheit der zu testenden Substanz.

Reportergene sind Gene oder Genfragmente, die für möglichst einfach nachweisbare Genprodukte kodieren, z. B. photometrisch durch Farbreaktionen. Häufig verwendete Reportergene sind das Gen für β-Galactosidase, das Gen für die alkalische Phosphatase, das Gen für Chloramphenicol-Acetyltransferase, das Gen für die Catechol-Dioxygenase, das Gen für das "green" oder "blue fluorescent protein" sowie verschiede-Sequenz aufweisendes ne Luciferase-Gene, die die Zellen zum Leuchten bringen können. Durch Vorschaltung eines geeigneten Kontrollelementes, z.B. einer Promotor-Enhancer Sequenz, die unter der Kontrolle eines bestimmten Transkriptionsfaktors oder einer bestimmten Signaltransduktionskaskade steht, kann beispielsweise die Aktivität des Transkriptionsfaktors bzw. der Kaskade anhand der Menge des exprimierten Genproduktes bestimmt werden.

Herkömmlicherweise werden derartige Reportergene in geeigneten Vektoren unter Vorschaltung der interessierenden Promotor-Enhancer Sequenz in die Zellen eingebracht. Zur Analyse der steroidalen Aktivität von Substanzen eignen sich - abhängig vom zu analysierenden NR - alle bekannten NR-Zielsequenzen. Beispiel einer solchen Vektors, ist der Vektor MMTV-Luciferase, der zur Messung der androgenen Wirkung von Substanzen eingesetzt wird.

Substanzen mit einem hormonellen Effekt, vorzugsweise einem androgenen/antiandrogenen Effekt, sind dann an der im Vergleich zu Versuchsansätzen ohne Beigabe der zu testenden Substanz erhöhten bzw. verminderten Expression des Reportergens erkennbar.

Zur Verwendung im Rahmen der vorliegenden Erfindung eignen sich, neben wildtyp EWS, EWS Derivate, welche die Aminosäuren 319 bis 656 der in Sequenz ID No. 1 beschriebenen Sequenz aufweisen, und hier besonders funktionelle EWS-Derivate, welche die Aminosäuren 319 bis 656 der in Sequenz ID No. 1 beschriebenen Sequenz aufweisen, die die Fähigkeit behalten haben, die Aktivität mindestens eines nukleären Rezeptors, insbesondere die von Androgenrezeptoren, zu modulieren oder diesen zumindest zu binden (in durch geeignete Methoden nachweisbarer und nicht zu vernachlässigender Weise - z.B. in Protein-Protein Interaktionsassays wie EMSA; der zuständige Fachmann kann hier differenzieren).

EWS und EWS kodierende Nukleinsäuren sind im Stand der Technik bereits bekannt (s.o.). Zur Verwendung im Rahmen der vorliegenden Erfindung ist vorzugsweise ein durch die Nukleinsäure gemäß Seq. ID No.1 kodiertes EWS, bzw. ein abgeleitetes Derivat geeignet (insbesondere ein funktionelles Derivat), welche die Aminosäuren 319 bis 656 der in Sequenz ID No. 1 beschriebenen Sequenz aufweisen. Besonders bevorzugt ist ein EWS Derivat, welches die Aminosäuren 319 bis 656 der in Seq. ID No. 1 beschriebene Sequenz aufweist, insbesondere ein Fragment, welches aus diesen Aminosäuren besteht.

Die Erfindung bezieht sich demgemäß auch auf die Verwendung von EWS bzw. dessen Derivaten welche die Aminosäuren 319 bis 656 der in Sequenz ID No. 1 beschriebenen Sequenz aufweisen zur Identifizierung und Charakterisierung von Substanzen, die die Aktivität des Androgenrezeptors, beeinflussen.

Das erfindungsgemäße Verfahren bzw. die erfindungsgemäße Verwendung der genannten Proteine bzw. Nukleinsäuren eignet sich insbesondere zur Analyse des hormonellen Effektes von Substanzen auf Androgenrezeptoren. Aufgrund der besonders gut charakterisierten Wirkung von EWS bzw. die obengenannten EWS-Derivaten auf den Androgenrezeptor, kommt dieser in besonders bevorzugten Ausführungsformen der Erfindung zum Einsatz.

Überdies kann EWS Verwendung als klinischer Indikator androgenbedingter Erkrankungen finden. Relevante androgenbedingte Erkrankungen sind z.B. Prostatakrebs, Glatzenbildung, Akne oder Hypogonadismus, sowie Androgenresistenzsyndrome, wie z.B. die testikuläre Feminisierung. Diese beruhen vermutlich auf Defekten im Co-Modulationsmechanismus zwischen Androgenrezeptor (AR) und EWS. Eine plausible diagnostische Möglichkeit bei Patienten mit derartigen Störungen bestünde somit in der Messung der relativen Raten von AR und EWS. Dies ist möglich durch Anwendung quantitativer Verfahren zur Messung der relativen Menge beider Moleküle in dem Zielgewebe bei dem jeweiligen Patienten.

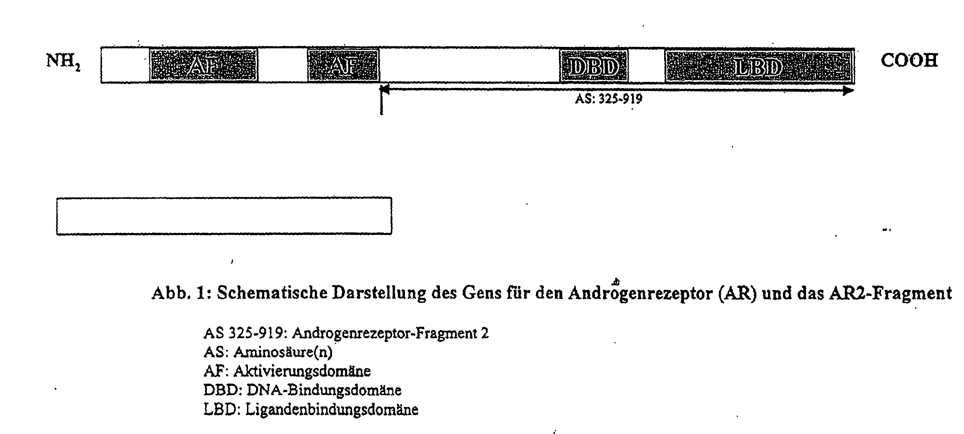

Die Erfindung wird nachfolgend anhand eines Beispiels unter bezugnahme auf die Abbildungen näher erläutert.
- Es stellen dar:: Abbildung 1 eine schematische Darstellung des Gens für den Androgenrezeptor (AR) und das AR2-Fragment,
Abbildung 2 eine schematische Darstellung des Gens für das Ewing-Sarkom-Protein (EWS),
Abbildung 3 die Sequenzen von EWS-Exonen und -Proteinen
Abbildung 4 die Co-Aktivierung des AR-Signals in SH-SY5Y-Zellen,
Abbildung 5 zeigt die Gewebsverteilung des EWS-Transkripts (Abb. 5a) und des AR-Transkripts (Abb. 5b).

### Beispiel 1:

### Verwendete Oligonukleotide:

Primer für die PCR-Amplifikation der Bibliotheks-Inserts:
   Act2c5050Eco: gattacgctagcttgggtgg (SEQ ID Nr. 3)
   Act2-4939Xho: gttgaagtgaacttggcgggg (SEQ ID Nr. 4)
Primer für die Amplifikation von EWS-cDNA in voller Länge:
   EWS-8-Sal: gggtcgacggacgttgagagaacgagg (SEQ ID Nr. 5)
   cESW-c2032-Eco: gggaattctgcggggtctctgcatctagtaggg (SEQ ID Nr. 6)
Sequenzierungs Primer:
   XII-139a1: gcttgggtggtcatatgg (SEQ ID Nr. 7)

### Verwendete Vektoren:

pACT2 (Genbank-Zugangsnummer U29899) für die Bibliothek;
pGBT9-Derivate für die Sonden: pGBT9rev und pGBT(+1)rev (Roder, K. H.; Wolf, S.S.; Schweizer, M., 1996, Analytical Biochemistry 241, 260-62);
pCR2.1Topo-Vektor (Firma Invitrogen) für die Klonierung von PCR-Fragmenten;
CMX-Vektor für die Expression in Säugetierzellen;
pAHluc für den Reportergen-Assay (enthält den MMTV-Promoter und ein Luciferase-Reportergen; Firma A. Cato);
pSGSAR (pSG5 mit dem humanen Gen für den Androgenrezeptor; Genbank-Zugangsnummer AAA51775).

### Verwendete Organismen:

- Hefestämme:: Y187 und PJ69-2A
- E.-coli-Stamm:: DH5α
- Säugetierzellen:: SH-SYSY (Deutsche Sammlung von Mikroorganismen und Zellkulturen GmbH (DSMZ): DSM ACC209);
PC3 (American type Culture Collection (ATCC): CRL-1435);
PC3AR: mit pSGSAR stabil transfizierte PC3 (Firma A. Cato, Karlsruhe, Deutschland).

Zur Identifizierung neuer Co-Modulatoren des Androgenrezeptors, wurde mit Hilfe des Hefe-Zweihybrid-Systems eine humane cDNA-Bibliothek ("Matchmaker" der Firma Clontech; Nr. HY4028AH) aus fetalem Gehirn mit drei verschiedenen Fragmenten des Androgenrezeptors (AR) als Sonde gescreent.

Hierzu wurde der Vektor pSG5AR, der die cDNA für den humanen AR enthält (Genbank AAA51775), mit Hilfe der. Endonuklease PstI so aufgespalten, dass drei verschiedene AR-DNA-Fragmente entstanden. Das kürzeste dieser Fragmente (AR4) kodiert für den N-Terminus des Rezeptors (AS 1-56), das mittlere (AR3) für den Mittelteil mit den Aktivierungsdomänen (AS 57-324) und das längste (AR2) für den C-Terminus (AS 325-918) mit der DNA- und der Ligandenbindungsdomäne (DBD und LBD; vergleiche Übersicht in Abbildung 1). AR2 wurde in den pGBT9(+1)rev-Vektor kloniert, nachdem dieser vorher mit Hilfe von Pstl linearisiert worden war.

Anschliessend erfolgte die Transformation des pGBT-Vektors, der das AR-Fragment enthielt, in den Hefestamm PJ69-2A. Die positiven Transformanten (Trp+) wurden nach den Anweisungen des Herstellers (Clontech) mit einer aus fetalem Gehirn gewonnenen cDNA-Bibliothek inkubiert (Humane Muldple-Tissue-CDNA (MTC), Panel II der Firma Clontech; Kat.: Nr. K1421-1). In Übereinstimmung mit den Anweisungen des Herstellers (Clontech) wurden 3x10⁶ Klone gescreent. Die positiven Klone wurden selektiert und nach den Anweisungen des Herstellers (Clontech) auf ihre β-Galaktosidase-Aktivität getestet. Die der Bibliothek entstammenden Inserts blauer Kolonien wurden mittels PCR und unter Verwendung der Primer Act2c5050Eco und Act 2-4939Xho direkt aus den Hefezellen amplifiziert.

Die PCR-Produkte wurden gelelektrophoretisch ihrer Länge nach aufgetrennt und mittels Spaltung durch Mspl weiter analysiert. Mindestens ein Exemplar jedes Restriktionsfragmentmusters wurde unter Verwendung von XII-139a1 als Sequenzprimer sequenziert. Die Sequenzen wurden mit der Genbank oder der Datenbank Incyte abgeglichen.

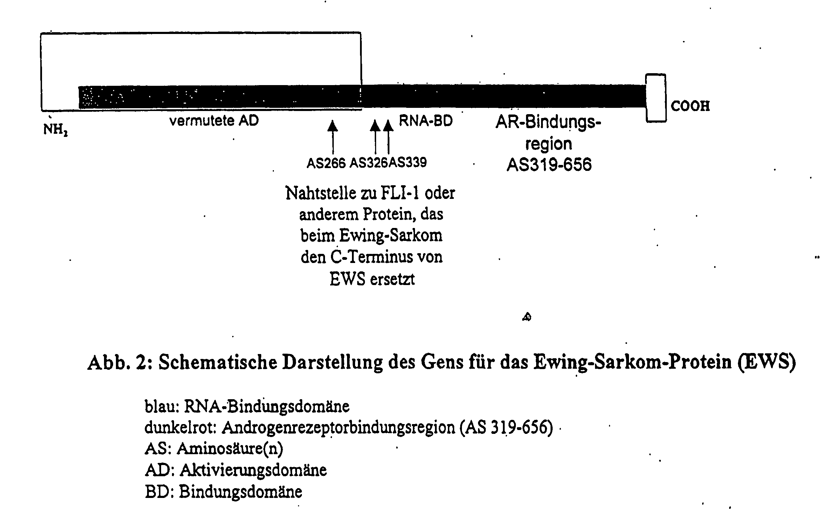

Eines der mehrfach identifizierten Inserts hatte eine Länge von 1500 bp und konnte durch Sequenzierung und Sequenzvergleich mit der Datenbank NCBJ als für den C-terminalen Anteil des menschlichen EWS (AS 319-656) kodierend identifiziert werden (Übersicht in Abbildung 2; Aminosäurensequenz in Abbildung 3).

Abbildung 3 zeigt cDNA Sequenz von humanem EWS mitsamt der abgeleiteten Aminosäuresequenz Dargestellt sind die Exone 1 bis 17. Die fettgedruckten Buchstaben bezeichnen das Fragment, das in Hefe-Zweihybrid-Systemen zu finden ist und an den AR-Abschnitt AS 325 bis AS 919 bindet. Unterstrichen sind die in den Splice-Varianten EWS1-b (durchgängig unterstrichen) bzw. EWS1-c (gepunktete Unterstreichung) fehlenden Sequenzbereiche.

Mittels PCR und unter Verwendung der Primer EWS-8-Sal und cEWS-c2032-Eco sowie Thymus- oder Milz-cDNA der Firma Clontech wurde EWS in voller Länge amplifiziert, wobei aus der Milz die vollständige codierende Region des Transkriptes und aus dem Thymus die Variante mit Exon 15 B anstelle von Exon 15 isoliert wurde. Die amplifizierte cDNA wurde dann mit EcoRI und Sal I in die Expressionscassette des Säugetierexpressionsvektors CMX kloniert.

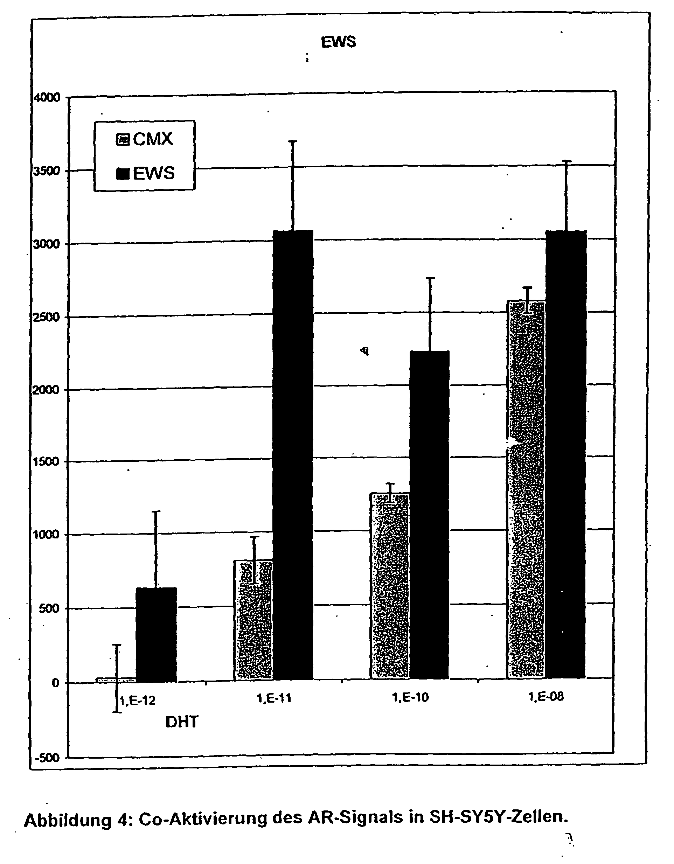

Wie aus dem in Abbildung 4 dargestellten Säulendiagramm hervorgeht, ist EWS nach transienter Transfektion in SH-SY5Y-Zellen fähig, eine starke Co-Aktivierung der AR-Signaltätigkeit herbeizuführen, insbesondere bei niedrigen Androgenkonzentrationen von 10⁻¹²-10⁻¹⁰ Mol. Hierzu wurden auf Reaktionsplatten mit jeweils sechs Reaktionsmulden SH-SY5Y-Zellen mit 0.75 µg eines Vektors, der die cDNA für den humanen Androgenrezeptor enthielt (pSG5AR), 1,5 µg des Reportergenkonstrukts pAHluc, das vor dem Luciferase-Gen den MMTV-Promotor enthält, und 1 µg des EWS-CMX-Vektors co-transfiziert. Die Transfektion erfolgte unter Verwendung von Lipofectin von der Firma Gibco BRC nach Angaben des Herstellers. Vierundzwanzig Stunden nach der Transfektion wurden die Zellen über Nacht mit unterschiedlichen Androgenmengen inkubiert. Die Zellen wurden mit einem herkömmlichen Lysispuffer lysiert, und die Luciferase-Aktivität im Lumistar-Luminometer von BMG Lab Technologies gemessen. Die EWS-CMX-Luciferase-Aktivitäten wurden mit den Kontrollaktivitäten verglichen (leerer CMX-Vektor). Die Mischung in jeder Mulde wurde in vier Mulden einer Mikrotiterplatte gemessen. Die Kontrollwerte der Substanz ohne DHT wurden subtrahiert. Die Standardbweichung ist in der Abbildung 4 durch Balken eingezeichnet.

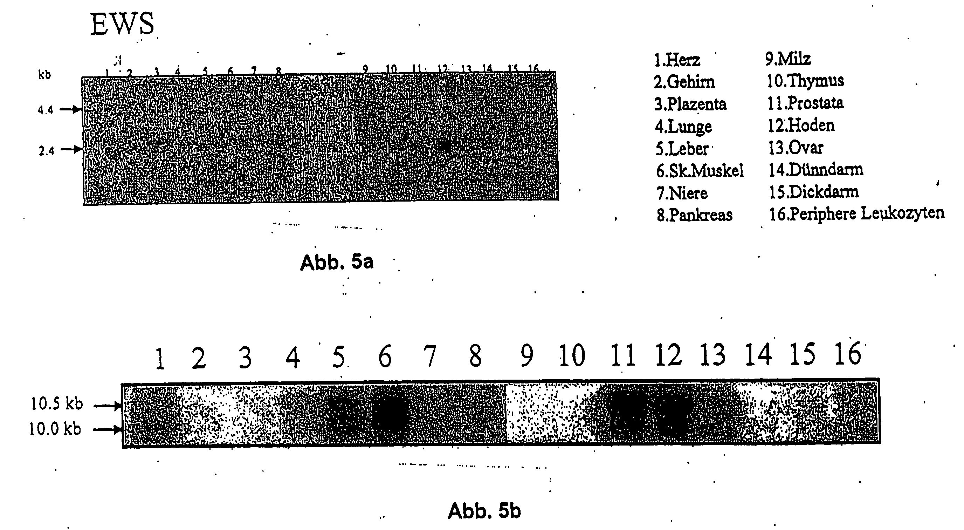

Die Gewebeverteilung des humanen EWS in normalem humanen Gewebe ist in Abbildung 5a anhand der dargestellten Autoradiographien ersichtlich. Hierzu wurde ein EWS-cDNA-Fragment, das für die Aminosäuren 244-656 von EWS kodiert, nach dem Megaprime^{™} DNA-Markierungssystem (Amersham Life Science) mit α-³²PdATP und dem Klenow-Fragment markiert. Das markierte Fragment wurde auf einer Nick-Säule (Pharmacia) nach Angaben des Herstelllers gereinigt und mit Human-Blots; Human Northern Blot (MTN) der Firma Clontech (Nr. 7760-1 und 7759-1) der Firma Clontech hybridisiert. Wie aus der Abbildung 5a hervorgeht, wird EWS-RNA vorwiegend in den Hoden exprimiert. Darüber hinaus sind in den meisten untersuchten Organen unterschiedliche EWS-Mengen nachweisbar.

Abb. 5b zeigt die Gewebeverteilung des humanen AR in normalem humanen Gewebe.

Aus den Abb. 5a und 5b lassen sich die Normexpressionen dieser beiden Proteine im Gewebe ermittlen.

Abbildung 4 zeigt die Co-Aktivierung des AR-Signals in SH-SY5Y-Zellen. In eine Reaktionsplatte mit sechs Reaktionsmulden wurden pro Mulde 1 µg Co-Aktivator, 1,5 µg MMTV-Luciferase und 0,75 µg pSG5AR-Plasmid gegeben. Die sechs Mischungen wurden in jeweils vier Vertiefungen einer Mikrotiterplatte überführt und dort gemessen. Die Fehlerabweichung ist als SD angegeben. Bei allen Signalen wurden die Werte der entsprechenden Kontrollen ohne DHT subtrahiert.

Abbildung 5a zeigt die Gewebsverteilung des EWS-Transkripts (Northem Blot MTN der Firma Clontech). Nach den Anweisungen des Herstellers (Amersham) erfolgte ein Zufallspriming des EWS-cDNA-Fragments, das für die Aminosäuren 244 bis 656 kodiert, und eine Markierung mit α-³²PdATP und dem Klenow-Fragment. Die Blots wurden mit der Sonde hybridisiert, gewaschen, auf einen Film übertragen und entwickelt.

## Patentansprüche

1. Verfahren zur Bestimmung des hormonellen Effektes von Substanzen mit den Schritten
a. Kontaktieren von EWS Protein oder eines die Aminosäuren 319 bis 656 der in Seq. ID No. 1 beschriebenen Sequenz aufweisendes Derivat davon mit dem Androgenrezeptor und einer Testsubstanz, und
b. Bestimmung des Einflusses der Testsubstanz auf die Bindung von EWS Protein oder des Derivats davon und dem Androgenrezeptor oder
c. Bestimmung des Einflusses der Testsubstanz auf die Liganden-induzierte Aktivität des Androgenrezeptors,
wobei das Verfahren nicht am menschlichen oder tierischen Körper vorgenommen wird.

2. Verfahren gemäß Anspruch 1, wobei mindestens ein Schritt im zellulären System abläuft mit den folgenden Schritten:
a. Zellen, die EWS Protein oder das Derivat davon und den Androgenrezeptor exprimieren, werden der zu testenden Substanz ausgesetzt,
b. Bestimmung des Einflusses der Substanz auf die Interaktion zwischen dem Androgenrezeptor und EWS Protein oder dem Derivat davon durch Messung der Protein-Protein-Interaktion oder der Protein-Protein-DNA Interaktion.

3. Verfahren gemäß Anspruch 1, wobei mindestens ein Schritt im zellulären System abläuft mit den folgenden Schritten:
a. Zellen, die EWS Protein oder das Derivat davon und den Androgenrezeptor exprimieren und mit einem Reportergenkonstrukt transfiziert sind, werden dem Liganden des Androgenrezeptors und der zu testenden Substanz ausgesetzt,
b. Bestimmung der Transkriptionsaktivität des Androgenrezeptors durch Messung der Reportergenaktivität und
c. Vergleich mit der Transkriptionsaktivität bei Durchführung der Schritte a und b in Abwesenheit der zu testenden Substanz.

4. Verfahren gemäß Anspruch 2 oder 3, **dadurch gekennzeichnet, dass** die Zellen eukaryontische Zellen, vorzugsweise Prostatazellen, Nervenzellen, Gliazellen, Fibroblasten, Blutzellen, Osteoblasten, Osteoklasten, Hepatozyten, Epithelzellen oder Muskelzellen sind.

## Claims

1. Method for determining the hormonal effect of substances with the steps of
a. contacting EWS protein or a derivative thereof having amino acids 319 to 656 of the sequence described in SEQ ID No. 1 with the androgen receptor and with a test substance, and
b. determining the influence of the test substance on the binding of EWS protein or the derivative thereof and the androgen receptor or
c. determining the influence of the test substance on the ligand-induced activity of the androgen receptor,
where the method is not carried out on the human or animal body.

2. Method according to Claim 1, where at least one step takes place in a cellular system with the following steps:
a. cells which express EWS protein or the derivative thereof and the androgen receptor are exposed to the substance to be tested,
b. determination of the influence of the substance on the interaction between the androgen receptor and EWS protein or the derivative thereof by measuring the protein-protein interaction or the protein-protein-DNA interaction.

3. Method according to Claim 1, where at least one step takes place in a cellular system with the following steps:
a. cells which express EWS protein or the derivative thereof and the androgen receptor and are transfected with a reporter gene construct are exposed to the ligand of the androgen receptor and to the substance to be tested,
b. determination of the transcription activity of the androgen receptor by measuring the reporter gene activity and
c. comparison with the transcription activity when steps a and b are carried out in the absence of the substance to be tested.

4. Method according to Claim 2 or 3, **characterized in that** the cells are eukaryotic cells, preferably prostate cells, nerve cells, glia cells, fibroblasts, blood cells, osteoblasts, osteoclasts, hepatocytes, epithelial cells or muscle cells.

## Revendications

1. Procédé de détermination de l'effet hormonal de substances, comprenant les étapes
a. de mise en contact d'une protéine EWS ou d'un dérivé de celle-ci présentant les acides aminés 319 à 656 de la séquence décrite dans SEQ. ID. NO 1 avec le récepteur d'androgène et une substance d'essai, et
b. de détermination de l'influence de la substance d'essai sur la liaison entre la protéine EWS ou son dérivé et le récepteur d'androgène, ou
c. de détermination de l'influence de la substance d'essai sur l'activité, induite par ligand, du récepteur d'androgène,
dans lequel le procédé n'est pas effectué sur un corps humain ou animal.

2. Procédé suivant la revendication 1, dans lequel au moins une étape se déroule dans un système cellulaire, ce procédé comprenant les étapes suivantes :
a. des cellules, qui expriment une protéine EWS ou le dérivé de celle-ci et le récepteur d'androgène, sont exposées à la substance à examiner,
b. une détermination de l'influence de la substance sur l'interaction entre le récepteur d'androgène et la protéine EWS ou son dérivé par mesure de l'interaction protéine-protéine ou de l'interaction protéine-protéine-ADN.

3. Procédé suivant la revendication 1, dans lequel au moins une étape se déroule dans un système cellulaire, ce procédé comprenant les étapes suivantes :
a. des cellules, qui expriment une protéine EWS ou son dérivé et le récepteur d'androgène et sont transfectées par une construction de gène rapporteur, sont exposées au ligand du récepteur d'androgène et à la substance à examiner,
b. une détermination de l'activité de transcription du récepteur d'androgène par mesure de l'activité du gène rapporteur, et
c. une comparaison avec l'activité de transcription lors de la mise en oeuvre des étapes a. et b. en présence de la substance à examiner.

4. Procédé suivant la revendication 2 ou 3, **caractérisé en ce que** les cellules sont des cellules eucaryotes, de préférence des cellules de prostate, des cellules nerveuses, des cellules gliales, des fibroblastes, des cellules sanguines, des ostéoblastes, des ostéoclastes, des hépatocytes, des cellules épithéliales ou des cellules musculaires.


### SEQUENZPROTOKOLL

<110> JENAPHARM GmbH & Co. KG
<120> Verfahren zur Bestimmung des hormonellen Effekts von Substanzen
<130> Pat 3684/11
<140>
   <141>
<160> 7
<170> PatentIn Ver. 2.1
<210> 1
   <211> 2390
   <212> DNA
   <213> Homo sapiens
<220> EWS
   <221> CDS
   <222> (44)..(2011)
<400> 1
   agagggagac ggacgttgag agaacgagga ggaaggagag aaa atg gcg tcc acg    55
                                                   Met Ala Ser Thr
                                                     1
   gat tac agt acc tat agc caa gct gca gcg cag cag ggc tac agt gct   103
   Asp Tyr Ser Thr Tyr Ser Gln Ala Ala Ala Gln Gln Gly Tyr Ser Ala
     5                  10                  15                  20
   tac acc gcc cag ccc act caa gga tat gca cag acc acc cag gca tat   151
   Tyr Thr Ala Gln Pro Thr Gln Gly Tyr Ala Gln Thr Thr Gln Ala Tyr
                    25                  30                  35
   ggg caa caa agc tat gga acc tat gga cag ccc act gat gtc agc tat   199
   Gly Gln Gln Ser Tyr Gly Thr Tyr Gly Gln Pro Thr Asp Val Ser Tyr
                40                  45                  50
   acc cag gct cag acc act gca acc tat ggg cag acc gcc tat gca act   247
   Thr Gln Ala Gln Thr Thr Ala Thr Tyr Gly Gln Thr Ala Tyr Ala Thr
            55                  60                  65
   tct tat gga cag cct ccc act ggt tat act act cca act gcc ccc cag   295
   Ser Tyr Gly Gln Pro Pro Thr Gly Tyr Thr Thr Pro Thr Ala Pro Gln
        70                  75                  80
   gca tac agc cag cct gtc cag ggg tat ggc act ggt gct tat gat acc   343
   Ala Tyr Ser Gln Pro Val Gln Gly Tyr Gly Thr Gly Ala Tyr Asp Thr
    85                  90                  95                 100
   acc act gct aca gtc acc acc acc cag gcc tcc tat gca gct cag tct    391
   Thr Thr Ala Thr Val Thr Thr Thr Gln Ala Ser Tyr Ala Ala Gln Ser
                   105                     110                 115
   gca tat ggc act cag cct gct tat cca gcc tat ggg cag cag cca gca    439
   Ala Tyr Gly Thr Gln Pro Ala Tyr Pro Ala Tyr Gly Gln Gln Pro Ala
               120                 125                     130
   gcc act gca cct aca aga ccg cag gat gga aac aag ccc act gag act    487
   Ala Thr Ala Pro Thr Arg Pro Gln Asp Gly Asn Lys Pro Thr Glu Thr
           135                 140                 145
   agt caa cct caa tct agc aca ggg ggt tac aac cag ccc agc cta gga    535
   Ser Gln Pro Gln Ser Ser Thr Gly Gly Tyr Asn Gln Pro Ser Leu Gly
       150                 155                     160
   tat gga cag agt aac tac agt tat ccc cag gta cct ggg agc tac ccc    583
   Tyr Gly Gln Ser Asn Tyr Ser Tyr Pro Gln Val Pro Gly Ser Tyr Pro
   165                 170                     175                 180
   atg cag cca gtc act gca cct cca tcc tac cct cct acc agc tat tcc    631
   Met Gln Pro Val Thr Ala Pro Pro Ser Tyr Pro Pro Thr Ser Tyr Ser
                   185                     190                 195
   tct aca cag ccg act agt tat gat cag agc agt tac tct cag cag aac    679
   Ser Thr Gln Pro Thr Ser Tyr Asp Gln Ser Ser Tyr Ser Gln Gln Asn
               200                 205                     210
   acc tat ggg caa ccg agc agc tat gga cag cag agt agc tat ggt caa    727
   Thr Tyr Gly Gln Pro Ser Ser Tyr Gly Gln Gln Ser Ser Tyr Gly Gln
           215                 220                     225
   caa agc agc tat ggg cag cag cct ccc act agt tac cca ccc caa act    775
   Gln Ser Ser Tyr Gly Gln Gln Pro Pro Thr Ser Tyr Pro Pro Gln Thr
       230                 235                     240
   gga tcc tac agc caa gct cca agt caa tat agc caa cag agc agc agc    823
   Gly Ser Tyr Ser Gln Ala Pro Ser Gln Tyr Ser Gln Gln Ser Ser Ser
   245                 250                     255                 260
   tac ggg cag cag agt tca ttc cga cag gac cac ccc agt agc atg ggt    871
   Tyr Gly Gln Gln Ser Ser Phe Arg Gln Asp His Pro Ser Ser Met Gly
                   265                     270                 275
   gtt tat ggg cag gag tct gga gga ttt tcc gga cca gga gag aac cgg    919
   Val Tyr Gly Gln Glu Ser Gly Gly Phe Ser Gly Pro Gly Glu Asn Arg
               280                 285                     290
   agc atg agt ggc cct gat aac cgg ggc agg gga aga ggg gga ttt gat    967
   Ser Met Ser Gly Pro Asp Asn Arg Gly Arg Gly Arg Gly Gly Phe Asp
           295                 300                     305
   cgt gga ggc atg agc aga ggt ggg cgg gga gga gga cgc ggt gga atg    1015
   Arg Gly Gly Met Ser Arg Gly Gly Arg Gly Gly Gly Arg Gly Gly Met
       310                 315                     320
   ggc agc gct gga gag cga ggt ggc ttc aat aag cct ggt gga ccc atg    1063
   Gly Ser Ala Gly Glu Arg Gly Gly Phe Asn Lys Pro Gly Gly Pro Met
   325                 330                 335                 340
   gat gaa gga cca gat ctt gat cta ggc cca cct gta gat cca gat gaa    1111
   Asp Glu Gly Pro Asp Leu Asp Leu Gly Pro Pro Val Asp Pro Asp Glu
                   345                 350                 355
   gac tct gac aac agt gca att tat gta caa gga tta aat gac agt gtg    1159
   Asp Ser Asp Asn Ser Ala Ile Tyr Val Gln Gly Leu Asn Asp Ser Val
               360                 365                 370
   act cta gat gat ctg gca gac ttc ttt aag cag tgt ggg gtt gtt aag    1207
   Thr Leu Asp Asp Leu Ala Asp Phe Phe Lys Gln Cys Gly Val Val Lys
           375                 380                 385
   atg aac aag aga act ggg caa ccc atg atc cac atc tac ctg gac aag    1255
   Met Asn Lys Arg Thr Gly Gln Pro Met Ile His Ile Tyr Leu Asp Lys
       390                 395                 400
   gaa aca gga aag ccc aaa ggc gat gcc aca gtg tcc tat gaa gac cca    1303
   Glu Thr Gly Lys Pro Lys Gly Asp Ala Thr Val Ser Tyr Glu Asp Pro
   405                 410                 415                 420
   ccc act gcc aag gct gcc gtg gaa tgg ttt gat ggg aaa gat ttt caa    1351
   Pro Thr Ala Lys Ala Ala Val Glu Trp Phe Asp Gly Lys Asp Phe Gln
                   425                 430                 435
   ggg agc aaa ctt aaa gtc tcc ctt gct cgg aag aag cct cca atg aac    1399
   Gly Ser Lys Leu Lys Val Ser Leu Ala Arg Lys Lys Pro Pro Met Asn
               440                 445                 450
   agt atg cgg ggt ggt ctg cca ccc cgt gag ggc aga ggc atg cca cca    1447
   Ser Met Arg Gly Gly Leu Pro Pro Arg Glu Gly Arg Gly Met Pro Pro
           455                 460                 465
   cca ctc cgt gga ggt cca gga ggc cca gga ggt cct ggg gga ccc atg    1495
   Pro Leu Arg Gly Gly Pro Gly Gly Pro Gly Gly Pro Gly Gly Pro Met
       470                 475                 480
   ggt cgc atg gga ggc cgt gga gga gat aga gga ggc ttc cct cca aga    1543
   Gly Arg Met Gly Gly Arg Gly Gly Asp Arg Gly Gly Phe Pro Pro Arg
   485                 490                 495                 500
   gga ccc cgg ggt tcc cga ggg aac ccc tct gga gga gga aac gtc cag    1591
   Gly Pro Arg Gly Ser Arg Gly Asn Pro Ser Gly Gly Gly Asn Val Gln
                   505                 510                 515
   cac cga gct gga gac tgg cag tgt ccc aat ccg ggt tgt gga aac cag    1639
   His Arg Ala Gly Asp Trp Gln Cys Pro Asn Pro Gly Cys Gly Asn Gln
               520                 525                 530
   aac ttc gcc tgg aga aca gag tgc aac cag tgt aag gcc cca aag cct    1687
   Asn Phe Ala Trp Arg Thr Glu Cys Asn Gln Cys Lys Ala Pro Lys Pro
           535                 540                 545
   gaa ggc ttc ctc ccg cca ccc ttt ccg ccc ccg ggt ggt gat cgt ggc      1735
   Glu Gly Phe Leu Pro Pro Pro Phe Pro Pro Pro Gly Gly Asp Arg Gly
       550                 555                 560
   aga ggt ggc cct ggt ggc atg cgg gga gga aga ggt ggc ctc atg gat      1783
   Arg Gly Gly Pro Gly Gly Met Arg Gly Gly Arg Gly Gly Leu Met Asp
   565                 570                 575                 580
   cgt ggt ggt ccc ggt gga atg ttc aga ggt ggc cgt ggt gga gac aga      1831
   Arg Gly Gly Pro Gly Gly Met Phe Arg Gly Gly Arg Gly Gly Asp Arg
                   585                 590                 595
   ggt ggc ttc cgt ggt ggc cgg ggc atg gac cga ggt ggc ttt ggt gga      1879
   Gly Gly Phe Arg Gly Gly Arg Gly Met Asp Arg Gly Gly Phe Gly Gly
               600                 605                 610
   gga aga cga ggt ggc cct ggg ggg ccc cct gga cct ttg atg gaa cag      1927
   Gly Arg Arg Gly Gly Pro Gly Gly Pro Pro Gly Pro Leu Met Glu Gln
           615                 620                 625
   atg gga gga aga aga gga gga cgt gga gga cct gga aaa atg gat aaa      1975
   Met Gly Gly Arg Arg Gly Gly Arg Gly Gly Pro Gly Lys Met Asp Lys
       630                 635                 640
   ggc gag cac cgt cag gag cgc aga gat cgg ccc tac tagatgcaga           2021
   Gly Glu His Arg Gln Glu Arg Arg Asp Arg Pro Tyr
   645                 650                 655
   gaccccgcag agctgcattg actaccagat ttatttttta aaccagaaaa tgttttaaat 2081
   ttataattcc atatttataa tgttggccac aacattatga ttattccttg tctgtacttt 2141
   agtatttttc accatttgtg aagaaacatt aaaacaagtt aaatggtagt gtgcggagtt 2201
   tttttttctt ccttctttta aaaatggttg tttaagactt taacaatggg aaccccttgt 2261
   gagcatgctc agtatcattg tggagaacca agagggcctc ttaactgtaa caatgttcat 2321
   ggttgtgatg tttttttttt ttttttaaaa taaaattcca aatgtttaat aaaaaaaaaa 2381
   aaaaaaaaa                                                         2390
<210> 2
   <211> 656
   <212> PRT
   <213> Homo sapiens
<400> 2
   Met Ala Ser Thr Asp Tyr Ser Thr Tyr Ser Gln Ala Ala Ala Gln Gln
   1 5 10 15
   Gly Tyr Ser Ala Tyr Thr Ala Gln Pro Thr Gln Gly Tyr Ala Gln Thr
   20 25 30
   Thr Gln Ala Tyr Gly Gln Gln Ser Tyr Gly Thr Tyr Gly Gln Pro Thr
   35 40 45
   Asp Val Ser Tyr Thr Gln Ala Gln Thr Thr Ala Thr Tyr Gly Gln Thr
   50 55 60
   Ala Tyr Ala Thr Ser Tyr Gly Gln Pro Pro Thr Gly Tyr Thr Thr Pro
   65 70 75 80
   Thr Ala Pro Gln Ala Tyr Ser Gln Pro Val Gln Gly Tyr Gly Thr Gly
   85 90 95
   Ala Tyr Asp Thr Thr Thr Ala Thr Val Thr Thr Thr Gln Ala Ser Tyr
   100 105 110
   Ala Ala Gln Ser Ala Tyr Gly Thr Gln Pro Ala Tyr Pro Ala Tyr Gly
   115 120 125
   Gln Gln Pro Ala Ala Thr Ala Pro Thr Arg Pro Gln Asp Gly Asn Lys
   130 135 140
   Pro Thr Glu Thr Ser Gln Pro Gln Ser Ser Thr Gly Gly Tyr Asn Gln
   145 150 155 160
   Pro Ser Leu Gly Tyr Gly Gln Ser Asn Tyr Ser Tyr Pro Gln Val Pro
   165 170 175
   Gly Ser Tyr Pro Met Gln Pro Val Thr Ala Pro Pro Ser Tyr Pro Pro
   180 185 190
   Thr Ser Tyr Ser Ser Thr Gln Pro Thr Ser Tyr Asp Gln Ser Ser Tyr
   195 200 205
   Ser Gln Gln Asn Thr Tyr Gly Gln Pro Ser Ser Tyr Gly Gln Gln Ser
   210 215 220
   Ser Tyr Gly Gln Gln Ser Ser Tyr Gly Gln Gln Pro Pro Thr Ser Tyr
   225 230 235 240
   Pro Pro Gln Thr Gly Ser Tyr Ser Gln Ala Pro Ser Gln Tyr Ser Gln
   245 250 255
   Gln Ser Ser Ser Tyr Gly Gln Gln Ser Ser Phe Arg Gln Asp His Pro
   260 265 270
   Ser Ser Met Gly Val Tyr Gly Gln Glu Ser Gly Gly Phe Ser Gly Pro
   275 280 285
   Gly Glu Asn Arg Ser Met Ser Gly Pro Asp Asn Arg Gly Arg Gly Arg
   290 295 300
   Gly Gly Phe Asp Arg Gly Gly Met Ser Arg Gly Gly Arg Gly Gly Gly
   305 310 315 320
   Arg Gly Gly Met Gly Ser Ala Gly Glu Arg Gly Gly Phe Asn Lys Pro
   325 330 335
   Gly Gly Pro Met Asp Glu Gly Pro Asp Leu Asp Leu Gly Pro Pro Val
   340 345 350
   Asp Pro Asp Glu Asp Ser Asp Asn Ser Ala Ile Tyr Val Gln Gly Leu
   355 360 365
   Asn Asp Ser Val Thr Leu Asp Asp Leu Ala Asp Phe Phe Lys Gln Cys
   370 375 380
   Gly Val Val Lys Met Asn Lys Arg Thr Gly Gln Pro Met Ile His Ile
   385 390 395 400
   Tyr Leu Asp Lys Glu Thr Gly Lys Pro Lys Gly Asp Ala Thr Val Ser
   405 410 415
   Tyr Glu Asp Pro Pro Thr Ala Lys Ala Ala Val Glu Trp Phe Asp Gly
   420 425 430
   Lys Asp Phe Gln Gly Ser Lys Leu Lys Val Ser Leu Ala Arg Lys Lys
   435 440 445
   Pro Pro Met Asn Ser Met Arg Gly Gly Leu Pro Pro Arg Glu Gly Arg
   450 455 460
   Gly Met Pro Pro Pro Leu Arg Gly Gly Pro Gly Gly Pro Gly Gly Pro
   465 470 475 480
   Gly Gly Pro Met Gly Arg Met Gly Gly Arg Gly Gly Asp Arg Gly Gly
   485 490 495
   Phe Pro Pro Arg Gly Pro Arg Gly Ser Arg Gly Asn Pro Ser Gly Gly
   500 505 510
   Gly Asn Val Gln His Arg Ala Gly Asp Trp Gln Cys Pro Asn Pro Gly
   515 520 525
   Cys Gly Asn Gln Asn Phe Ala Trp Arg Thr Glu Cys Asn Gln Cys Lys
   530 535 540
   Ala Pro Lys Pro Glu Gly Phe Leu Pro Pro Pro Phe Pro Pro Pro Gly
   545 550 555 560
   Gly Asp Arg Gly Arg Gly Gly Pro Gly Gly Met Arg Gly Gly Arg Gly
   565 570 575
   Gly Leu Met Asp Arg Gly Gly Pro Gly Gly Met Phe Arg Gly Gly Arg
   580 585 590
   Gly Gly Asp Arg Gly Gly Phe Arg Gly Gly Arg Gly Met Asp Arg Gly
   595 600 605
   Gly Phe Gly Gly Gly Arg Arg Gly Gly Pro Gly Gly Pro Pro Gly Pro
   610 615 620
   Leu Met Glu Gln Met Gly Gly Arg Arg Gly Gly Arg Gly Gly Pro Gly
   625 630 635 640
   Lys Met Asp Lys Gly Glu His Arg Gln Glu Arg Arg Asp Arg Pro Tyr
   645 650 655
<210> 3
   <211> 20
   <212> DNA
   <213> synthetisch
<220> Primer
   <400> 3
   gattacgcta gcttgggtgg 20
<210> 4
   <211> 21
   <212> DNA
   <213> synthetisch
<220> Primer
   <400> 4
   gttgaagtga acttggcggg g 21
<210> 5
   <211> 27
   <212> DNA
   <213> synthetisch
<220> Primer
   <400> 5
   gggtcgacgg acgttgagag aacgagg 27
<210> 6
   <211> 33
   <212> DNA
   <213> synthetisch
<220> Primer
   <400> 6
   gggaattctg cggggtctct gcatctagta ggg 69
<210> 7
   <211> 18
   <212> DNA
   <213> synthetisch
<220> Primer
   <400> 7
   gcttgggtgg tcatatgg 17